**Europäisches Patentamt**

**European Patent Office**

**Office européen des brevets**

(19)

Veröffentlichungsnummer: **0 267 446**

**A1**

(12) **EUROPÄISCHE PATENTANMELDUNG**

(21) Anmeldenummer: **87114888.8**

(22) Anmeldetag: **13.10.87**

(51) Int. Cl.4: **A61M 25/00 , A61M 23/00**

(30) Priorität: **16.10.86 DE 3635183**

(43) Veröffentlichungstag der Anmeldung:
**18.05.88 Patentblatt 88/20**

(84) Benannte Vertragsstaaten:
**AT BE CH DE ES FR GB GR IT LI LU NL SE**

(71) Anmelder: **Medizintechnik Wedel GmbH & Co. KG**
**Feldstrasse 150**
**D-2000 Wedel/Holstein(DE)**

(72) Erfinder: **Dams, Klaus, Prof.Dr.med.**
**Parkstrasse 1a**
**D-2000 Hamburg 52(DE)**
Erfinder: **List, Gunther,**
**Prof.Dr.rer.nat.Dipl.-Phys.**
**Opm. Blockhorn 3g**
**D-2000 Schenefeld(DE)**
Erfinder: **Subke, Henning, Prof.Dr.-Ing.**
**Mittelstrasse 9**
**D-2000 Schenefeld(DE)**

(74) Vertreter: **Struck, Willi, Dr.-Ing.**
**Friedrich-Ebert-Strasse 10f**
**D-2080 Pinneberg(DE)**

(54) **Mobile Darmsonde.**

(57) Die Erfindung betrifft eine mobile Sonde für die Einführung in den Körper, insbes. in die Darmpartien von Menschen oder Tieren, bei der in an sich bekannter Weise am vorderen Ende eines aus dem Körper herausführenden biegsamen Schlauches (10) ein in die Darmpartie einzuführender Sondenkörper (20) angeordnet ist und bezweckt eine Sonde zu - schaffen, die in verhältnismäßig kurzer Zeit auch in die tieferen Darmpartien einführbar ist.

Erfindungsgemäß wird dazu vorgeschlagen, den Sonden körper (20) mit einer aktiv betätigbaren Vorschubeinrichtung, beispielsweise einer zwischen zwei Grundplatten (25, 26) verschieblichen auf einem Führungsstab (24) geführten trägen Masse (27), die durch Stromimpulse bewegt wird, zu versehen.

Fig. 1

**EP 0 267 446 A1**

## Mobile Darmsonde

Die Erfindung betrifft eine mobile Sonde für die Einführung in den Körper, insbes. in die Darmpartien von Menschen oder Tieren, bei der in an sich bekannter Weise am vorderen Ende eines aus dem Körper herausführenden biegsamen Schlauches ein in die Darmpartie einzuführender Sondenkörper angeordnet ist.

Der Darmverschluß gehört zu einer der gefährlichsten Erkrankungen, der jährlich viele Menschen zum Opfer fallen. Durch einen Darmverschluß tritt vor dem Hindernis ein Sekretstau auf, der zur Fäulnisbildung und zur Vergiftungsgefahr in den Nieren führt. In akuten Fällen kommt es dann darauf an, diesen Sekretstau insbesondere im überdehnten Dünndarm möglichst schnell abzubauen, was in bekannter Weise durch Absaugung des Sekretes im gestauten Magen-Darmtrakt geschieht.

Diese Absaugung stößt jedoch bis heute auf große Schwierigkeiten, da die bekannten passiven Entlastungssonden,wie die Sonde nach Miller-Abbott,in der Regel kurzfristig nur bis in den Magen, allenfalls in den oberen Zwölffingerdarm geführt werden können. Dort bleiben sie infolge Peristaltiklähmung liegen und erreichen die prallgefüllten tieferen Dünndarmabschnitte nicht mehr. So ist man bis heute gezwungen, unter Zeitnot den nicht optimal vorbereiteten Patienten einer Notoperation mit allen ihren Risiken zuzuführen.

Diese Mängel sollen durch die vorliegende Erfindung, durch die die Aufgabe gelöst werden soll, eine Sonde zu schaffen, die in verhältnismäßig kurzer Zeit auch in die tieferliegenden Darmpartien einführbar ist, vermieden werden.

Zur Lösung dieser Aufgabe wird bei einer Sonde der eingangs genannten Art erfindungsgemäß vorgeschlagen, den Sondenkörper mit einer aktiv betätigbaren Vorschubeinrichtung zu versehen.

Die Vorschubeinrichtung kann darin bestehen, daß im Sondenkörper eine elektrische Spule angeordnet ist, die eine auf einem Führungsstab zwischen zwei Grundplatten geführte träge Masse umgibt. Dieser Spule werden Stromimpulse durch elektrische Leitungen aus einer außerhalb des Körpers angeordneten Stromquelle zugeführt, die durch den mit dem Sondenkörper verbundenen Schlauch hindurchgeführt sind. Die in Vorschubrichtung vordere Grundplatte kann dabei als Prallplatte ausgebildet sein und vor der hinteren Grundplatte soll eine Feder angeordnet sein.

Die Achse des Führungsstabes für die träge Masse kann in Richtung der Achse des Sondenkörpers liegen, sie kann aber auch gegenüber dieser Achse geneigt sein.

Die Vorschubeinrichtung kann auch aus einem im Sondenkörper angebrachten Zylinder bestehen, in dem ein federnder Faltenbalg druckdicht einerseits mit dem Zylinder und andererseits mit einer trägen Masse verbunden ist, in dessen Innenraum eine Flüssigkeit mit wechselndem Druck einführbar ist, so daß die Masse wechselnd verschiebbar ist. Zur Schaffung eines Schwingungssystems kann im Innenraum des Faltenbalges noch ein gasgefüllter elastischer Ballon angebracht sein.

Um die Druckflüssigkeit gleichzeitig als Spülflüssigkeit verwenden zu können, soll vor dem Zylinder in der Flüssigkeitsleitung ein Oberdruckventil mit einer anschließenden Spülflüssigkeitsleitung zum Kopf des Sondenkörpers vorgesehen sein.

Weiterhin kann der Sondenkörper als im wesentlichen in seiner Längserstreckung elastisch dehnbarer Körper ausgebildet sein, an dessen Enden im wesentlichen in Umfangsrichtung aufweitbare Preßkörper angebracht sein sollen. Die Ausdehnung des Sondenkörpers und die Aufweitung der Preßkörper soll mittels Druckmedium gesteuert erfolgen. Der Sondenkörper kann dabei aus einer Spiralfeder bestehen, die von einer Plastikhaut flüssigkeitsdicht umhüllt ist, oder auch aus einem oder mehreren Faltenbälgen, die mit einem Schutzüberzug versehen sind. Es hat sich als zweckmäßig erwiesen, in der Plastikhaut oder zwischen den Faltenbälgen Einschnürungen oder besondere biegeweiche Stellen vorzusehen. Auch bei dieser Ausführungsform kann das Druckmedium gleichzeitig als Spülflüssigkeit durch Anordnung eines Überdruck-bzw. Überströmventiles dienen, die den Spülöffnungen im Kopf des Sondenkörpers zugeführt sind.

Alle Leitungen wie Stromleitungen für die Spule, Flüssigkeitsleitungen für die Zuführung von Druckflüssigkeit oder Spülflüssigkeit sollen in dem mit dem Sondenkörper verbundenen Schlauch verlegt sein. Ebenfalls die mit den Absaugeöffnungen verbundenen Absaugeleitungen.

Auf der beiliegeden Zeichnung sind verschiedene Ausführungsbeispiele der Erfindung dargestellt. Dabei zeigen

Fig. 1 einen Sondenkörper mit elektrodynamischem Antrieb,

Fig. 2 einen Sondenkörper mit hydrodynamischen Antrieb,

Fig. 3 einen Sondenkörper mit hydro-statischem Antrieb und

Fig. 4 eine abgewandelte Ausführung eines solchen Sondenkörpers in Teilansicht.

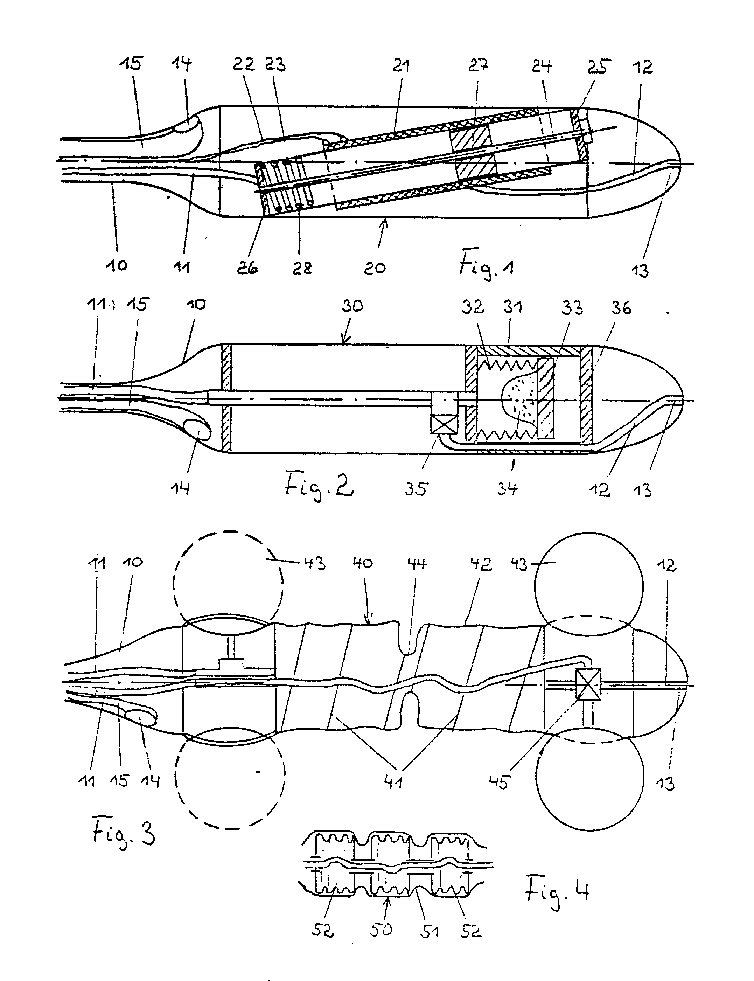

Bei dem in Fig. 1 dargestellten Sondenkörper mit elektrodynamischen Antrieb wird im Innern des Sondenkörpers 20 eine träge Masse 27 auf einem Stab 24 geführt. Durch das Magnetfeld einer umgebenden Spule 21 wird die Masse mit konstanter Beschleunigung in Richtung Spitze bewegt und - schlägt schließlich mit der höchsten Geschwindigkeit gegen die Grundplatte 25 des Sondenkörpers. Der erfährt dadurch eine hohe Kraftspitze in Richtung einer Längsachse, was ihn befähigt, sich auch durch Gebiete hohen Reibungswiderstandes im Darminneren zu bewegen.

Dieser Vorgang wiederholt sich periodisch, unterstützt durch die Feder 28 an der Grundplatte 26, wobei über Spulenstrom und Taktfrequnz die Vortriebsleistung eingestellt werden kann.

Um das Festfahren in einer Darmschlinge zu verhindern, kann der Stab 24 auch, wie dargestellt, geneigt zur Sondenlängsachse angeordnet sein. Auf diese Weise wird die Sonde eine - schraubenförmige Vorwärtsbewegung ausführen und so die Möglichkeit erhalten, sich aus einem Hindernis selbst zu befreien.

Am Kopf der Sonde befindet sich eine Öffnung 13 für die Spü 1 flüssigkeit, die den Darminhalt ggf. verdünnt, damit er am Fuß der Sonde durch die Öffnung 14 abgesaugt werden kann.

Bei dem in Fig. 2 gezeigten Sondenkörper ist im Inneren des Sondenkörpers 30 ebenfalls eine träge Masse 33, die jetzt den Abschlub eines Fluidvolumens bildet, das von einer federbelasteten Faltenmembrane 32 und einer Grundplatte mit dem Schlauchende gebildet wird. Wird das Fluidvolumen aufgefüllt, so bewegt sich die Masse 33 nach vorn und schlägt auf eine Platte 36 an der Sondenstruktur. Hierdurch wird ebenfalls eine Kraftsp itze in Längsrichtung erzeugt, die die im Darm auftretende Reibung überwindet. Fügt man in das Fluidvolumen einen gasgefüllten elastischen Ballon ein, so ergibt sich ein schwingungsfähiges System, bei dem die Masse durch einen pulsierenden Druckverlauf in der Zuleitung zum Schwingen angeregt wird.

Auch hier kann die Bewegungsrichtung schräg zur Sonderlängs achse orientiert sein, um ein Festfahren der Sonde in einer Darmfalte zu verhindern.

Bei Verwendung eines Fluids zum Antrieb der Sonde kann dasselbe Fluid als Spülflüssigkeit verwendet werden, wodurch sich eine Leitung einsparen läßt. Durch die Festlegung des Druckniveaus wird der Durchsatz der Spülflüssigkeit über ein federbelastetes Druckventil 35 festgelegt, während Frequenz und Amplitue der überlagerten Druckschwankungen die Vortriebsleistung bestimmen. Außerdem wird durch die Druckschwankungen in der Schlauchleitung deren Reibung herabgesetzt.

Bei dem in Fig. 3 gezeigten hydrostatischen ANtrieb besteht der Sonderkörper 40 aus einer Spiralfeder 41, über die eine Plastikhaut gezogen und verschweißt wurde. Durch eine Druckerhöhung im Innern des Körpers streckt er sich und schiebt den Kopf vor. Ist die größte Streckung erreicht. bläht sich am Kopf ein ringförmiger Preßkörper 43 auf, der sich an die Darmwand anpreßt. Durch Druckabsenkung im Innern des Körpers 40 zieht sich die Spiralfeder 41 wieder zusammen und holt das Sondenende nach; danach bläht sich auch hier ein ringförmiger Preßkörper 42 auf, der das Sondenende an der Darmwand abstützt.

Der gesamte Vorgang wiederholt sich periodisch, so daß sich die Sonde raupenförmig vorwärts bewegt.

Um das Durchlaufen einer Darmfalte zu ermöglichen, befindet sich in der Mitte eine biegeweiche Einschnürung 44.

Statt aus einer Plastikhaut kann der Sondenkörper 50 auch, wie in Fig. 4 angedeutet ist, aus mehreren elastisch verbundenen, durch einen elastischen Oberzug 51 abgedeckten Faltenbälgen 52 bestehen.

Die erfindungsgemäße Sonde bietet auch die Möglichkeit Sensoren für Temperatur, Leitfähigkeit usw. an bestimmte Stellen in den Darm zu bringen. Auch Einrichtungen zur Entnahme von Gewebeproben oder zur optischen Inspektion oder auch Schläuche zum Einspritzen von Kontrastmittel oder dergl. lassen sich mit der erfindungsgemäßen Sonde transportieren.

## Ansprüche

1. Mobile Sonde für die Einführung in den Körper, insbes. in die Darmpartien von Menschen oder Tieren, bei der in an sich bekannter Weise am vorderen Ende eines aus dem Körper herausführenden biegsamen Schlauches ein in die Darmpartie einzuführender Sondenkörper angeordnet ist, dadurch gekennzeichnet, daß der Sondenkörper (20, 30, 40) mit einer aktiv betätigbaren Vorschubeinrichtung versehen ist.

2. Sonde nach Anspruch 1, dadurch gekennzeichnet, daß die Vorschubeinrichtung eine in einer in Längserstreckung des Sondenkörpers (20) im Sondenkörper angeordneten umgebenden elektrischen Spule (21), deren Stromführungsleitungen (22, 23) durch den mit dem Sondenkörper verbundenen Schlauch (10) zu einer außerhalb des Körpers angeordneten Stromquelle geführt sind, auf einem Führungsstab (24) zwischen zwei Grundplatten (25, 26) verschieblich geführte träge Masse (27) ist.

3. Sonde nach Anspruch 1 und 2, dadurch gekennzeichnet, daß die in Vorschubrichtung des Sondenkörpers (20) vordere Grundplatte (25) als Prallplatte ausgebildet ist und vor der hinteren Grundplatte (26) eine Feder (28) angeordnet ist.

4. Sonde nach Anspruch 1 bis 3, dadurch gekennzeichnet, daß die Achse der Vorschubeinrichtung bzw. der Spule (21) oder des Führungsstabes (24) gegenüber der Längsachse des Sondenkörpers (20) geneigt ist.

5. Sonde nach ANspruch 1, dadurch gekennzeichn et, daß die Vorschubeinrichtung ein im Sondenkörper (30) angebrachter Zylinder (31) ist, in dem eine am vorderen Ende eines an ihrem hinteren Ende mit dem Zylinder druckdic ht verbundenen federnden Faltenbalges (32), in dessen Innenraum über eine im mit dem Sondenkörper verbundenen Schlauch (10) geführte Flüssigkeitsleitung (11) eine Flüssigkeit mit wechselndem Druck einführbar ist, angebrachte träge Masse (33) verschieblich gelagert ist.

6. Sonde nach Anspruch 5, dadurch gekennzeichnet, daß im Innenraum des Faltenbalges (32) ein gasgefüllter elastischer Ballon (34) angebracht ist.

7. Sonde nach Anspruch 5 und 6, dadurch gekennzeichnet, daß vor dem Zylinder (31) in der Flüssigkeitsleitung (11) ein Oberdruckventil (35) mit einer anschließenden Spülflüssigkeitsleitung (12) vorgesehen ist.

8. Sonde nach Anspruch 1, dadurch gekennzeichnet, daß der Sondenkörper (40) als im wesentlichen in seiner Längserstreckung mittels eines gesteuert unter Druck zugeführten Mediums elastisch dehnbarer Körper ausgebildet ist, an dessen vorderem und hinterem Ende wahlweise durch gesteuert unter Druck zugeführtes Medium im wesentlichen in Umfangsrichtung aufweitbare Preßkörper (43) angeordnet sind.

9. Sonde nach Anspruch 8, dadurch gekennzeichnet, daß der Sondenkörper (40) durch eine mit einer Plastikhaut (42) flüssigkeitsdicht überzogene Spiralfeder (41) gebildet ist.

10. Sonde nach Anspruch 8 und 9, dadurch gekennzeichnet, daß die Plastikhaut (42) mit einer oder mehreren biegeweichen Einschnürungen (44) versehen ist.

11. Sonde nach Anspruch 8, dadurch gekennzeichnet, daß der Sondenkörper (50) aus einem oder mehreren elastisch miteinander verbundenen, gegebenenfalls mit einem elastischen Oberzug (51) versehenen, Faltenbälgen (52) besteht.

12. Sonde nach Anspruch 8 bis 11, dadurch gekennzeichnet, daß in der Zuführungsleitung (11) für die Druckflüssigkeit ein Oberdruckventil (45) mit einer anschließenden Spülflüssigkeitsleitung (12) angebracht ist.

13. Sonde nach Anspruch 1 bis 12, dadurch gekennzeichnet, daß am Kopf des Sondenkörpers (20, 30, 40) Spülöffnungen (13) vorgesehen sind, denen die Spülflüssigkeit durch im Innenraum des Schlauches (10) verlegte Flüssigkeitsleitungen (11) zugeführt wird.

14. Sonde nach Anspruch 1 bis 13, dadurch gekennzeichnet, daß am Sondenkörper (20, 30, 40) mit durch den Schlauch (10) geführten Absaugeleitungen (15) verbundene Absaugeöffnungen (14) angeordnet sind.

0 267 446

Fig. 1

Fig. 2

Fig. 3

Fig. 4

| | **EINSCHLÄGIGE DOKUMENTE** | | EP 87114888.8 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| X | US - A - 4 176 662 (R.E. FRAZER) | 1,8,11 | A 61 M 25/00 |
| | * Fig. 1,3; Spalte 1, Zeilen 15-19; Spalte 2, Zeile 56 - Spalte 3, Zeile 34 * | | A 61 M 23/00 |
| | -- | | |
| X | US - A - 3 895 637 (D.S.J. CHOY) | 1 | |
| A | * Gesamt; insbesondere Spalte 3, Zeilen 51-61; Spalte 4, Zeilen 6-32, 39-56 * | 8,11 | |
| | -- | | |
| X | DE - A1 - 3 111 497 (W. SCHUBERT) | 1 | |
| A | * Patentansprüche 1,2,13,15,18; Seite 14, 1. Absatz - Seite 16, 2. Absatz; Fig. 1,2 * | 12,13 | |
| | -- | | |
| X | AT - B - 366 904 (H. PRISTAUZ) | 1 | **RECHERCHIERTE SACHGEBIETE (Int. Cl 4)** |
| A | * Fig. 2,4,6; Seite 2, Zeile 52 - Seite 3, Zeile 43; Seite 3, Zeile 52 - Seite 4, Zeile 9; Seite 4, Zeilen 30-39 * | 8,11 | A 61 B 1/00 |
| | | | A 61 M 23/00 |
| | | | A 61 M 25/00 |
| | ---- | | |

Der vorliegende Recherchenbericht wurde für alle Patentansprüche erstellt.

| Recherchenort | Abschlußdatum der Recherche | Prüfer |
|---|---|---|
| WIEN | 24-02-1988 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82